# EUROPEAN PATENT APPLICATION

(11) **EP 4 844 073 A1**
(43) Date of publication of application: **25.11.2026**
(21) Application number: 26176347.8
(22) Date of filing: 04.05.2026
(51) Int. Cl.: C12Q 1/6825, C12Q 1/6869

(54) **SYSTEMS AND METHODS FOR PROXIMITY-INHIBITED DNA SEQUENCING BY REPEATEDSUBSEQUENCES**

(30) Priority: 19.05.2025 US 202519211853
(71) Applicant: Robert Bosch GmbH, 70442 Stuttgart (DE)
(72) Inventor: Kornbluth, Mordechai, Brighton, Massachusetts, 02135 (US); Johnson, Christopher, San Carlos, California, 94070 (US); Pilsl, Sebastian, 70191 Stuttgart (DE)

(57) **Abstract**

Systems and methods for detecting more than one portion of a biomolecule during a single pass through a sensor are provided. Systems may include a device and a readout interface. The device may include at least one electrode and at least one channel that may receive a labeled linear biomolecule having a series of repeated units each separated by blocks of known length that shift where a particular portion of the linear biomolecule is at the top at one position and at the opposite side of the linear biomolecule at another position. Methods may include providing a sample including at least one biomolecule and generating a linear biomolecule comprising a series of repeated units each separated by the blocks of known length. The linear biomolecule may be labeled and delivered to a device having at least one electrode and at least one channel for detection of the label.

## Description

### TECHNICAL FIELD

The present disclosure relates to systems and methods of sensing molecules having key dimensions incommensurate with the size of a sensing zone.

### SEQUENCE LISTING

The xml file RBPA0546PUS_Sequence_Listing.xml of size 1.51 KB created May 16, 2025 filed herewith, is hereby incorporated by reference.

### BACKGROUND

Biosensor MEMS (Micro-Electro-Mechanical Systems) are used in medical diagnostics and biological research. Such systems provide a highly sensitive and accurate means of detecting and analyzing biomolecules. Certain biomolecules may have dimensions that are incommensurate with the size of the sensing zone of a biosensor. The size of a sensing zone may therefore determine the portion of a biomolecule that may be arranged within the sensing zone at a given time during a pass through the sensor.

### SUMMARY

In at least an aspect, a system for detecting more than one portion of a biomolecule during a single pass through a sensor is provided. The system may comprise a device including at least one electrode and at least one channel sized to receive a linear biomolecule having at least one label. The linear biomolecule may comprise a series of repeated units each separated by blocks of known length. The blocks of known length may shift the structure of the linear biomolecule so that at least one moiety of the linear biomolecule on a first side of the linear biomolecule at a first position is on a second side of the linear biomolecule at a second position. The system may also include a readout interface having at least one connection to the device. The readout interface may detect a signal generated by the at least one label and sensed by the at least one electrode. The at least one channel may be at least one nanochannel having diameter(s) of less than 1 µm. The at least one label may be at least one redox moiety capable of generating electrical signal(s). The signal generated by the at least one label may be sensed via proximity(ies) of the at least one label to the at least one electrode at a known potential. The device may further include a sensing zone proximate to two electrodes separated by a dielectric of size 1 to 10 nm. The linear biomolecule may be a polynucleotide. The series of repeated units may be concatemerized copies of the polynucleotide sequence. The blocks of known length may be a sequence of length N or a sequence of length N+X. The blocks of known length may include an identifier sequence.

In at least another aspect, a method of detecting more than one portion of a biomolecule during a single pass through a sensor is provided. The method may comprise providing a sample including at least one biomolecule, the at least one biomolecule comprising a single unit; attaching at least one block of known length to the at least one biomolecule; repeating the single unit of the at least one biomolecule and the at least one block of known length more than one time to generate a linear biomolecule comprising a series of repeated units each separated by the blocks of known length, wherein the blocks of known length shift the structure of the linear biomolecule so that at least one moiety of the linear biomolecule on a first side of the linear biomolecule at a first position is on a second side of the linear biomolecule at another position; modifying the at least one moiety on the linear biomolecule with at least one label to generate a labeled linear biomolecule; delivering the labeled linear biomolecule to a device including at least one electrode and at least one channel; and detecting a signal generated by the at least one label and the at least one electrode. The at least one biomolecule may be at least one polynucleotide. The series of repeated units may be concatemerized copies of the at least one polynucleotide. The blocks of known length may be polynucleotide sequences of length N or length N+X. The blocks of known length may include an identifier sequence. The at least one label may be at least one redox moiety capable of generating an electrical signal(s). The at least one polynucleotide may be fragmented via tagmentation or sonication prior to attaching the blocks of known length to the at least one biomolecule to produce a tunable distribution of lengths of the at least one polynucleotide in the sample.

In at least another aspect, a method of detecting more than one portion of a polynucleotide during a single pass through a sensor is provided. The method may comprise providing a sample including initial template polynucleotides; fragmenting the initial template polynucleotides in the sample; blunt end repairing and tailing the fragmented polynucleotides; ligating a hairpin adapter onto each end of each of the blunt end repaired and tailed polynucleotides to form a circularized dumbbell structure; amplifying the circularized dumbbell structure using at least two non-complementary redox-modified dNTPs having distinguishable signals to generate a single-stranded labeled linear polynucleotides having multiple concatemerized copies of the initial template polynucleotides separated by the hairpin adapters; delivering the labeled linear polynucleotide to a device including at least one electrode and at least one channel; and detecting a signal generated by the label and the at least one electrode. The circularized dumbbell structure may be amplified via rolling circle amplification. The initial template polynucleotides may be fragmented via tagmentation or sonication prior to attaching the hairpin adapters to the at least one biomolecule to produce a tunable distribution of lengths of the at least one polynucleotide in the sample. The plurality of the initial template polynucleotides may be fragmented via restriction digestion to generate polynucleotides of known length.

### BRIEF DESCRIPTION OF THE DRAWINGS

Figure 1 illustrates a plot showing that the higher the portion of a biomolecule is above the electrode, the smaller the signal.
Figure 2 illustrates an exemplar method for preparing DNA molecules for detection.
Figure 3 illustrates examples of hairpin adapters to finetune the orientation of a DNA molecule within the sensing zone of a nanogap.
Figure 4 is a system for detecting multiple portions of a biomolecule during a single pass through a sensor.
Figures 5A and 5B illustrate graphs of simulation data for a redox-labeled biomolecule according to an embodiment.
Figure 6 is a graph illustrating calculations of electron transmission through different redox-type molecules as a function of tunneling length.
Figures 7A-7D are graphs of profiles for redox-labeled DNA strands unrotated (=0°), or rotated 90°, 180°, or 270°. Figure 7A illustrates the profile for ferrocene. Figure 7B illustrates the profile for ferrocene with a carboxylic acid end group. Figure 7C illustrates the profile for methylene blue. Figure 7D illustrates the profile for boron dipyrromethene (BODIPY).

### DETAILED DESCRIPTION

As required, detailed embodiments of the present disclosure are disclosed herein; however, it is to be understood that the disclosed embodiments are merely exemplary of the disclosure that may be embodied in various and alternative forms. The figures are not necessarily to scale; some features may be exaggerated or minimized to show details of particular components. Therefore, specific structural and functional details disclosed herein are not to be interpreted as limiting, but merely as a representative basis for teaching one skilled in the art to variously employ the present disclosure.

Except in the examples, or where otherwise expressly indicated, all numerical quantities in this description indicating amounts of material or conditions of reaction and/or use are to be understood as modified by the word "about". The first definition of an acronym or other abbreviation applies to all subsequent uses herein of the same abbreviation and applies mutatis mutandis to normal grammatical variations of the initially defined abbreviation; and, unless expressly stated to the contrary, measurement of a property is determined by the same technique as previously or later referenced for the same property.

Unless indicated otherwise, all technical and scientific terms used herein have the same meaning as commonly understood by one of ordinary skill in the art to which the present disclosure belongs.

This disclosure is not limited to the specific embodiments and methods described below, as specific components and/or conditions may, of course, vary. Furthermore, the terminology used herein is used only for describing particular embodiments and is not intended to be limiting in any way.

As used in the specification and the appended claims, the singular form "a," "an," and "the" comprise plural referents unless the context clearly indicates otherwise. For example, reference to a component in the singular is intended to comprise a plurality of components.

The terms "or" and "and" can be used interchangeably and can be understood to mean "and/or".

The term "comprising" is synonymous with "including," "having," "containing," or "characterized by." These terms are inclusive and open-ended and do not exclude additional, unrecited elements or method steps.

The phrase "consisting of" excludes any element, step, or ingredient not specified in the claim. When this phrase appears in a clause of the body of a claim, rather than immediately following the preamble, it limits only the element set forth in that clause; other elements are not excluded from the claim as a whole.

The phrase "consisting essentially of' limits the scope of a claim to the specified materials or steps, plus those that do not materially affect the basic and novel characteristic(s) of the claimed subject matter.

The terms "comprising", "consisting of", and "consisting essentially of" can be alternatively used. When one of these three terms is used, the presently disclosed and claimed subject matter can include the use of either of the other two terms.

Integer ranges may explicitly include all intervening integers. For example, the integer range 1-10 explicitly includes 1, 2, 3, 4, 5, 6, 7, 8, 9, and 10. Similarly, the range 1 to 100 includes 1, 2, 3, 4. . . . 97, 98, 99, 100. Similarly, when any range is called for, intervening numbers that are increments of the difference between the upper limit and the lower limit divided by 10 can be taken as alternative upper or lower limits. For example, if the range is 1.1. to 2.1 the following numbers 1.2, 1.3, 1.4, 1.5, 1.6, 1.7, 1.8, 1.9, and 2.0 can be selected as lower or upper limits. In the specific examples set forth herein, concentrations, temperature, and reaction conditions (e.g., pressure, pH, etc.) can be practiced with plus or minus 50 percent of the values indicated rounded to three significant figures. In a refinement, concentrations, temperature, and reaction conditions (e.g., pressure, pH, etc.) can be practiced with plus or minus 30 percent of the values indicated rounded to three significant figures of the value provided in the examples. In another refinement, concentrations, temperature, and reaction conditions (e.g., pH, etc.) can be practiced with plus or minus 10 percent of the values indicated rounded to three significant figures of the value provided in the examples.

In any examples set forth herein, concentrations, temperature, and reaction conditions (e.g., pressure, pH, flow rates, etc.) can be practiced with plus or minus 50 percent of the values indicated rounded to or truncated to two significant figures of the value provided in the examples. In a refinement, concentrations, temperature, and reaction conditions (e.g., pressure, pH, flow rates, etc.) can be practiced with plus or minus 30 percent of the values indicated rounded to or truncated to two significant figures of the value provided in the examples. In another refinement, concentrations, temperature, and reaction conditions (e.g., pressure, pH, flow rates, etc.) can be practiced with plus or minus 10 percent of the values indicated rounded to or truncated to two significant figures of the value provided in the examples.

The terms "polynucleotide", "nucleotide", "nucleotide sequence", "nucleic acid", "polynucleic acid", and "oligonucleotide" may be used interchangeably in one or more embodiments this disclosure. They refer to a polymeric form of nucleotides of any length, either deoxyribonucleotides or ribonucleotides, or analogs thereof. Polynucleotides may have any three-dimensional structure, and may perform any function, known or unknown. The following are nonlimiting examples of polynucleotides: single-, double-, or multi-stranded DNA or RNA, genomic DNA, cDNA, DNA -RNA hybrids, or a polymer comprising purine and pyrimidine bases or other natural, chemically or biochemically modified, non-natural, or derivatized nucleotide bases. The terms "polynucleotide" and "nucleic acid" may include, as applicable to the embodiment being described, single-stranded (such as sense or antisense) and double-stranded polynucleotides. A polynucleotide may comprise one or more modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component. The term "base" refers to a single nucleotide in one or more embodiments.

The terms "complementarity" or "complement" may refer to the ability of a nucleic acid to form hydrogen bond(s) with another nucleic acid sequence by either traditional Watson-Crick or other non-traditional types. A percent complementarity indicates the percentage of residues in a nucleic acid molecule which can form hydrogen bonds (e.g., Watson-Crick base pairing) with a second nucleic acid sequence (e.g., 4, 5, and 6 out of 6 being 66.67%, 83.33%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 40%, 50%, 60%, 62.5%, 70%, 75%, 80%, 85%, 90%, 95%, 97%, 98%, 99%, or 100%, or percentages in between over a region of 4, 5, 6, 7, and 8 nucleotides, or refers to two nucleic acids that hybridize under stringent conditions.

The processes, methods, or algorithms disclosed herein can be deliverable to and/or implemented by a processing device, controller, or computer, which can include any existing programmable electronic control unit or dedicated electronic control unit. Similarly, the processes, methods, or algorithms can be stored as data and instructions executable by a controller or computer in many forms including, but not limited to, information permanently stored on non-writable storage media such as ROM devices and information alterably stored on writeable storage media such as floppy disks, magnetic tapes, CDs, RAM devices, and other magnetic and optical media. The processes, methods, or algorithms can also be implemented in an executable software object. Alternatively, the processes, methods, or algorithms can be embodied in whole or in part using suitable hardware components, such as Application Specific Integrated Circuits (ASICs), Field-Programmable Gate Arrays (FPGAs), state machines, controllers or other hardware components or devices, or a combination of hardware, software and firmware components.

Biosensor MEMS (Micro-Electro-Mechanical Systems) are used in medical diagnostics and biological research. Such systems provide a highly sensitive and accurate means of detecting and analyzing biomolecules. One of the key components of biosensor MEMS is the use of nanochannels, which are tiny channels on the order of dozens to hundreds of nanometers in size, to manipulate and move biomolecules such as DNA for example, often within water. These nanochannels allow for precise control and manipulation of biomolecules, enabling them to be positioned next to nanoelectrodes for measurement.

The interaction between the biomolecule and the nanoelectrode is then measured by the flow of current, providing valuable information about the properties and the behavior of the biomolecule. This technology has wide-ranging applications in fields such as medical diagnostics, drug development, and genetic research. The use of nanochannels in biosensor MEMS may be used for highly sensitive and precise detection and analysis of biomolecules.

A challenge in sequencing DNA molecules via nanochannels is that in a one-dimensional geometry, the sensing zone may be smaller than the size of the DNA molecule. Alternatively, or in addition, it may be the case that the sensing zone is longer than a single nucleotide on the DNA molecule. It may therefore be difficult to sense a biomolecule whose key dimensions are incommensurate with the size of the sensing zone.

The size of a sensing zone may therefore determine the portion of a biomolecule that may be arranged within the sensing zone at a given time during a pass through the sensor. Given a sensing device including an electrode of a width of at least 10 nm and a nanochannel of a width 10 to 1000 nm, and a biomolecule such as DNA in that channel, for example, the sensing zone may not be large enough to encompass both the top and bottom of the biomolecule. In one or more embodiments, provided herein are systems and methods for sensing more than one portion of a biomolecule during a single pass through the sensor. For example, the systems and methods in one or more embodiments provided herein may be utilized to sequence an entire DNA molecule in a single pass through the sensor.

In an ideal scenario, a biomolecule with velocity vx passing by an electrode of sensing zone d at height zm may generate a signal of a Gaussian lineshape of the following form, where t is time and z0 and J0 are both parameters. $j \left(t\right) = {J}_{0} exp \left(-\frac{2}{{z}_{0}}\sqrt{{d}^{2} + {z}_{m}^{2}}\right) exp \left(-\frac{16 {t}^{2} {v}_{x}^{2}}{{d}^{2}}\right)$

The prefactor's exponential dependence on zm means that for zm~d it will be critical to control the height zm for an appropriate signal magnitude.

Simultaneously, the width of the peak is proportional to vx/d, lending to velocity control, which can be given by an effective viscosity µ, which relates the velocity to the applied force as follows: vx=Fzm/µA, where F is the force of the fluid on the biomolecule, µ is the effective viscosity, and A is the effective area.

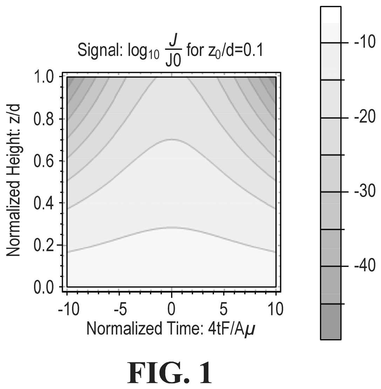

Converting to dimensionless units and assuming a value of z0/d=0.1, gives the plot shown in Figure 1. The portions that are at larger values of z are exponentially smaller in signal. In this way, the higher the portion of a biomolecule is above the electrode, the smaller the signal. Given the helical structure of a DNA strand, labeled nucleotides are differentially distributed from the sensor with corresponding signal intensity. Nucleotides passing closer to the sensor will have higher signal intensity, and nucleotides passing farther form the sensor may not generate a signal above a background threshold.

In one or more embodiments, systems and methods for detecting multiple portions of a biomolecule during a single pass through a sensor are provided herein. The biomolecule may be a polynucleotide. For example, the biomolecule may be a DNA molecule. The biomolecule may be a DNA molecule with additional tag moieties to enhance the signal. The biomolecule may be a DNA molecule derived from a single stranded RNA molecule. Double stranded DNA may be generated from single stranded RNA via RT-PCR or template switching-based methods, for example, depending on whether the RNA sequence is known or unknown. A DNA molecule may also be referred to as a DNA strand or as a polynucleotide. Methods for detecting multiple portions of a biomolecule during a single pass through a sensor may include ligating a sequence of a defined length to a DNA molecule to be sequenced. Depending on the number of nucleotides in the sequence of defined length, the ligation of the sequence of defined length to the DNA molecule may alter the angular orientation of a particular portion of the DNA molecule with respect to the sensor. The sequence may be of length N, meaning that the sequence has N nucleotides. The sequence of length N may be ligated to the DNA molecule more than once. By repeating the DNA sequence according to a known sequence length, N for example, the angular orientation of one or more portions of the DNA molecule may be altered with respect to the sensor. In this way, the bottom and top portions of the DNA molecule may be sequentially measured.

For example, a standard B-type DNA strand has a diameter of approximately 2 to 2.3 nm. The nucleotides (base pairs) of the DNA strand are spaced approximately 0.3 to 0.34 nm apart, and the helical pitch is approximately 3.3 to 3.5 nm, for a total pitch of 10-11 base pairs. This value may differ based on the particular conditions and experiment.

Given that the typical pitch of a standard B-type DNA strand is approximately 10.5 base pairs, then the sequence of a DNA biomolecule that contains one sequence of N equaling 21 base pairs will be approximately identical on each pass, because the angular orientation will be the same. However, if the one sequence of N is 15 base pairs, then subsequent measurements will have a rotation of approximately 180°, allowing the base pairs that used to be on the top to now be on the bottom.

In one or more embodiments, a method for detecting two or more portions of a biomolecule during a single pass through a sensor may include providing a sample having at least one biomolecule and attaching at least one sequence of length N to the at least one biomolecule. The at least one biomolecule may be a polynucleotide. The at least one biomolecule may be a DNA molecule, for example. The sample may include a plurality of DNA molecules of varying length. For example, at least some of the DNA molecules may have a length with an even number of nucleotides and other DNA molecules may have a length with an odd number of nucleotides. Prior to ligating the sequence of length N onto the DNA molecules in the provided sample, the DNA molecules in the sample may be fragmented by various mechanical or enzymatic methods. If sonication or tagmentation is used, for example, the generated DNA fragment lengths may be distributed around a tunable mean length. Alternatively, restriction digestion and PCR may be used to produce DNA fragments with known lengths. The length of the DNA molecules after ligation of the sequence of N nucleotides may thus be a mixture of sequences with an odd or an even number of nucleotides depending on how the DNA was fragmented.

Given a sample with DNA molecules of varying lengths, the defined sequence that may be ligated onto these random strands must be able to produce a desired rotational shift after amplification such that all of the nucleotides may be addressed with the sensor. In one or more embodiments, a method for detecting two or more portions of a biomolecule during a single pass through a sensor may include providing a sample of fragmented DNA molecules and ligating two different sequences of defined length onto a DNA molecule. For example, a first defined sequence of length N and a second defined sequence of length N+X may be ligated onto a DNA molecule. X may be from 1 to 21. Including sequences of defined length N and sequences of defined length N+1 in the ligation reaction may produce a shift of either 0, 1, or 2 nucleotides depending on the random ligation of the defined sequences (N or N+1) to the DNA molecule. Including sequences of defined length N and sequences of defined length N+5 in the ligation reaction may produce a shift of either 0, 5, or 10 nucleotides depending on the random ligation of the defined sequences (N or N+5) to the DNA molecule. A sequence of defined length N+21 may induce a full rotation. The shift in the structure of the linear biomolecule induced by the ligated sequences may cause at least one moiety of the linear biomolecule on a first side of the linear biomolecule at a first position to be on a second side of the linear biomolecule at a second position.

In various embodiments, two different defined sequences may be ligated onto the DNA molecules where the length of the ligated sequences is a distribution of length N, N+X, and N+2X, where X is between 1 and 21. By defining X, the shift per rotation that occurs on the subset of defined sequences that has the additional X bases ligated may be set. In some embodiments, N and/or X may be quasirandom, obeying some distribution with a known distribution function, mean and/or width. N and/or X may include a detectable and identifiable sequence that may be used to determine the beginning and/or end of the DNA molecule onto which the defined sequences of N, N+X, and/or N+2X have been ligated. This sequence may be referred to as an identifier sequence. The identifier sequences may be used to determine which defined sequences were ligated onto the DNA molecules.

In one or more embodiments, a method for detecting multiple portions of a biomolecule during a single pass through a sensor may further include labeling at least two non-complementary nucleotides with distinct labels having distinguishable signals on a DNA molecule to be detected. For example, Adenine and Guanine nucleotides may be labeled with labels having distinguishable signals, so that each Adenine and each Guanine nucleotide may be detected by the sensor. The labels may be electroactive moieties. The electroactive moieties may also be referred to as redox moieties or redox labels. The redox labels may be incorporated into the DNA molecule to be detected nucleotide by nucleotide in a nucleotide-specific manner so that the signals can be read out accurately. The redox labels may be incorporated into the DNA molecule to be detected by utilizing DNA library preparation methods that allow for: (i) accurate incorporation of redox labels; (ii) full sequence reconstruction; and (iii) consensus calling.

Redox labels may be incorporated into a DNA molecule to be detected by directly pre-labeling deoxynucleotide triphosphates (dNTPs) with the electroactive labels and incorporating them via rolling circle amplification or primer extension for example. Redox-modified dNTPs may only be incorporated into new strands via distinct polymerase enzymes. Polymerase enzymes capable of incorporating redox-modified dNTPs include Vent (exo-), SD Bioron, Therminator, KodXL, Klenow, Bst, and others.

Full sequence reconstruction may be achieved by incorporation of at least two non-complementary redox-modified dNTPs that are electrochemically distinguishable from each other. For example, redox-modified dCTP and dTTP/dUTP may be incorporated into a DNA molecule to be detected. Similarly, dATP and dGTP may be incorporated into a DNA molecule to be detected. Incorporating at least two non-complementary redox dNTPs may be referred to as a dual-label strategy. Where only two nucleotides are redox modified, both DNA strands (sense and antisense) must be sequenced to resolve the full DNA sequence. Additionally, sense and anti-sense strands must be connected physically. The sense and anti-sense strands of a redox-labeled DNA molecule to be detected may be physically connected via hairpin adapters or covalent linkage of at least one end. If at least three nucleotides are redox modified, one DNA strand would be sufficient to fully resolve the DNA sequence. Moreover, full sequence reconstruction may be achieved, when all four canonical nucleobases are redox-modified.

Using this approach according to one or more embodiments, limited base calling coverage for a single DNA passage may be achieved. Full sequence recovery may be achieved by collecting multiple readings of the same DNA molecule, therefore enabling consensus calling. Multiple readings of the same DNA molecule may be achieved by sequencing a single concatemerized DNA molecule that includes multiple copies of the DNA sequence to be detected.

An example of a library preparation strategy that may be used to prepare a redox-labeled DNA molecule that includes multiple copies of the DNA sequence to be detected is "rolling circle library" preparation. "Rolling circle" library preparation physically connects the sense and antisense strands of a double-stranded DNA molecule (dsDNA) on both ends and allows a full reconstruction plus simultaneous consensus calling of the sequence of a single redox-labeled DNA molecule containing sense and antisense strand information in multiple repeats. Furthermore, rolling circle library preparation may render the use of unique molecular identifiers (UMIs) unnecessary.

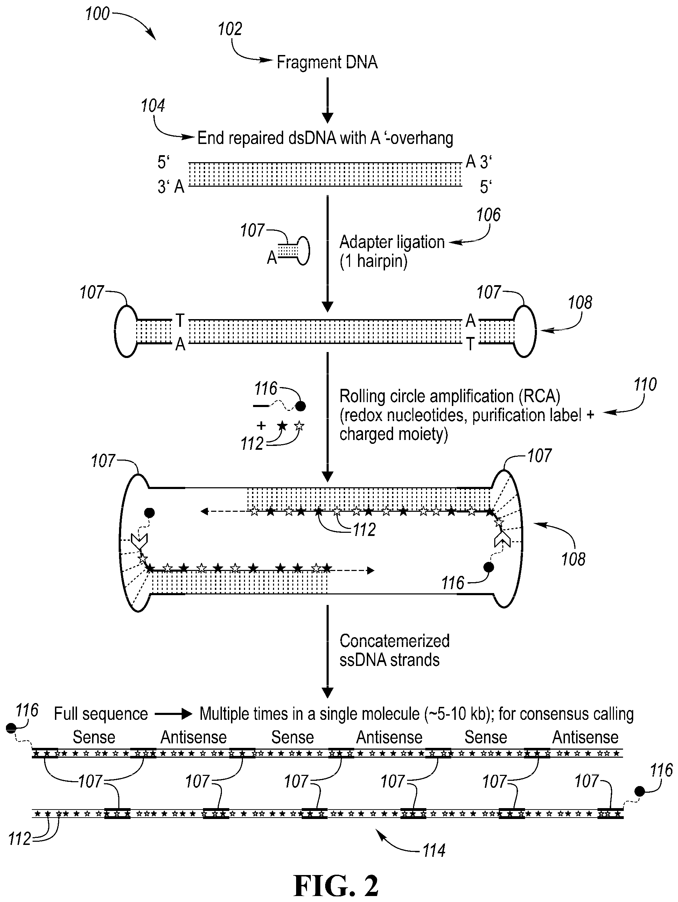

Figure 2 illustrates a method 100 for preparing DNA molecules to be detected. In one or more embodiments, DNA molecules to be detected (also referred to as template DNA) may be prepared for detection by first fragmenting the DNA molecules 102 by various options. If sonication or tagmentation is used, for example, the length of the fragmented DNA molecules may be distributed around a tunable mean length. Alternatively, restriction digestion and PCR may be used to produce DNA fragments with known lengths.

Next, the fragmented template DNA may be blunt end repaired 104 and dA-tailed to be ligated 106 with dT-tailed hairpin adapters 107 (also referred to as hairpins), so that a circularized dumbbell structure 108 is formed. The generated dumbbell structures 108 may vary in length, depending on the method of fragmentation used and the size of the hairpin adapters. Correctly ligated products may be isolated from by-products for example via optional exonuclease-treatment specific to free 3' and/or 5'-ends. In various embodiments, a rolling circle amplification (RCA) 110 may occur directly with redox-labeled nucleotides 112 using capable polymerases with strand displacement (SD) activity like SD Bioron, Therminator, Bst3.0, Vent (exo-), etc. The products of the rolling circle amplification may directly be sequenced after synthesis or subsequent purification. In other embodiments, rolling circle amplification may be performed with native nucleotides to amplify a new template which may be used as a template for the incorporation of redox nucleotides. The template may serve as a template for the incorporation of redox nucleotides in a primer extension reaction using a polymerase not necessarily requiring SD activity like Therminator, KodXL, Vent (exo-), Klenow etc. for example. In each case multiple, concatemerized copies 114 of the initial template DNA are located on a single single-stranded (ssDNA) molecule, optionally with or without hybridization to its unmodified reverse complementary DNA strand. This strategy only unlocks the preparation of redox-labeled ssDNA for Sequencing by electronic tunneling (SBET), either in a single-or double stranded format.

In one or more embodiments, the primers used for redox-label incorporation either during the RCA or afterward may be labeled with a purification label 116 for positive selection. The purification label 116 may be a biotin molecule. For example, the primers may be biotinylated for positive selection using streptavidin purification. In this case, positively selected redox-labeled ssDNA may be specifically subjected to sequencing. In another embodiment, the hairpin adapters 107 which are used in ligation 106 may encode a known and clearly detectable number of redox nucleotide repeats. For example, the hairpin adapters 107 may include 5-10 redox nucleotides in a row or 5-20 alternating redox nucleotides in a row. It is important to ensure that the repeats hybridize correctly. For example, it is important to ensure that correct loop formation and correct annealing occur so that ligation may take place. The corresponding redox-nucleotides become incorporated during the subsequent reactions after ligation 106 and they may be used as checkpoint regions to detect the beginning and the ending of the sequencing of each repetition of the DNA molecule of interest. In other embodiments, hairpin adapters 107 with different lengths may be simultaneously subjected to the ligation reaction. This enables the finetuning of the orientation of the nascent DNA strands after each repetition.

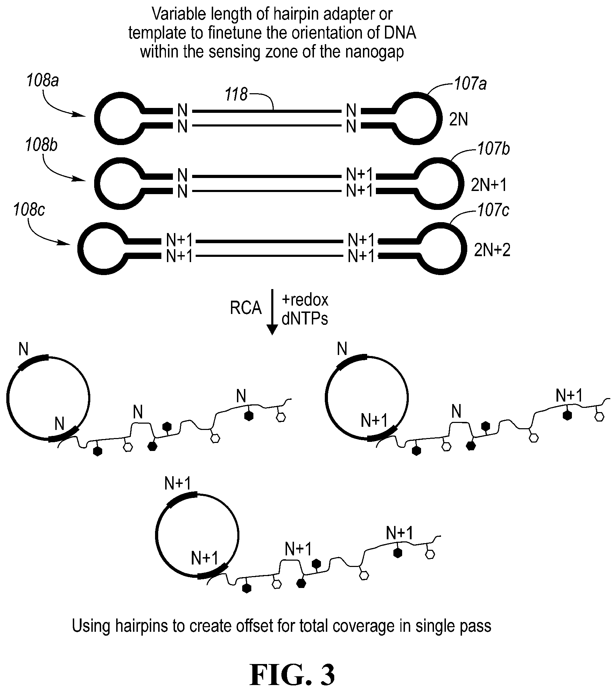

In other embodiments, hairpin adapters 107 with different lengths may be simultaneously subjected to the ligation reaction. This enables the finetuning of the orientation of the nascent DNA strands after each repetition. Figure 3 illustrates examples of hairpin adapters 107a and 107b to finetune the orientation of a DNA molecule within the sensing zone of a nanogap. Two hairpins of length N 107a may be ligated on to either end of a fragmented DNA molecule 118 to form a circularized dumbbell structure 108a. One hairpin of length N 107a may be ligated onto one end of a fragmented DNA molecule 118 and one hairpin of length N+1 107b may be ligated onto the other end of the fragmented DNA molecule 118 to form a circularized dumbbell structure 108b. Two hairpins of length N+1 107b may be ligated onto either end of a fragmented DNA molecule 118 to form a circularized dumbbell structure 108c. The dumbbell structures 108a, 108b, and 108c may differ in length from each other due to the difference in length of the hairpin adapters 107a, 107b ligated onto the fragmented DNA molecules 118. The difference in hairpin length may create offset for total coverage of the sequence of a DNA molecule in a single pass.

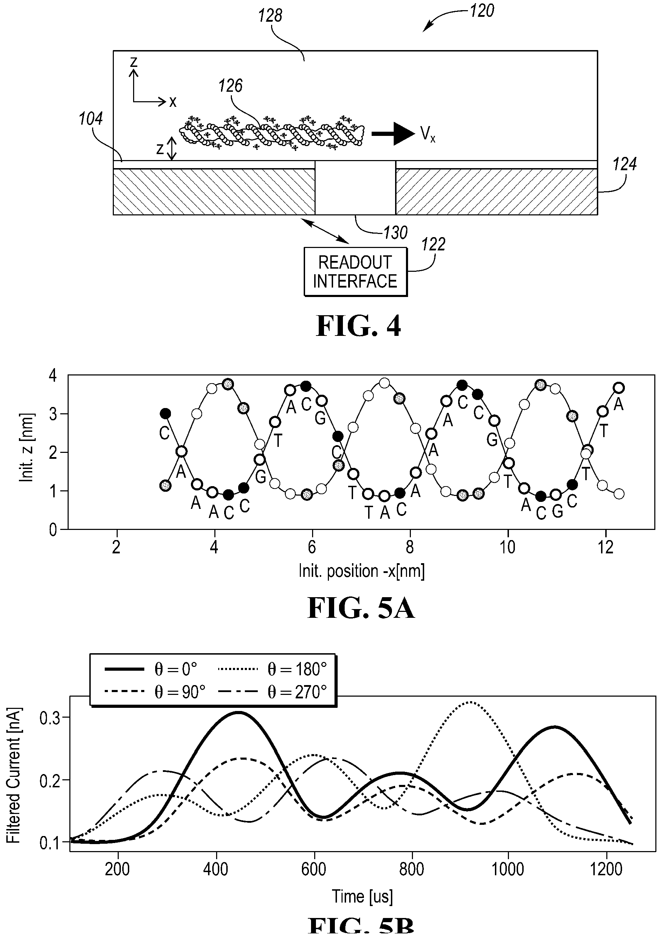

In one or more embodiments, a system for detecting multiple portions of a biomolecule during a single pass through a sensor is provided. The biomolecule may have a label. As illustrated in Figure 4, the system may include a device 120 and a readout interface 122. The readout interface 122 may detect a signal generated by the label. The device 120 may include at least one electrode 124. The electrode 124 may detect a linear biomolecule 126. The linear biomolecule 126 may be a polynucleotide. For example, the linear biomolecule 126 may be a DNA molecule. The device may additionally include at least one channel 128. The channel 128 may contain a solvent (not shown) and a linear biomolecule 126 to be detected. The channel 128 may be a nanochannel. The nanochannel may be <1 µm in diameter. Alternatively, the channel 128 may be from 1 to 999 µm in diameter. The channel 128 may also be millimeter-sized. The channel 128 may be adjacent to the at least one electrode 124. The device 120 may additionally include nanofluidics (not shown) and at least one connection to the readout interface 122. The device 120 may additionally include a partition between the two electrodes 130. The partition 130 may be a dielectric of a size from 2-10 nm. The dielectric may be flanked by two electrodes. The dielectric may be adjacent to a sensing zone which is a portion of the space within the channel. A linear biomolecule 126 may be detected in the sensing zone through proximity to one or more electrodes at known potential, optionally separated by a dielectric of size 2-10 nm. The linear biomolecule 126 may be passed through the channel 128 adjacent to the electrode 124 at a constant velocity of 0.1 microns per second to 1 mm per second.

The linear biomolecule 126 may be repeated over a known length. For example, the linear biomolecule 126 may be a series of repeated units. The units may each be separated by a block of known length. The presence of the blocks of known length in between the units may shift the structure of the linear biomolecule 126 such that moieties that are on top of the linear biomolecule 126 at one position are at the opposite side of the linear biomolecule 126 at another position. For example, the linear biomolecule 126 may be a DNA strand where the series of repeating units are multiple, concatemerized copies of an initial template DNA sequence on a single-stranded DNA molecule. The initial template DNA sequence may be an unidentified sequence that may need to be determined. The copies of the initial template DNA sequence may be separated by a repeated DNA sequence of known length (blocks). The repeats of the sequence of known length may shift the structure of the linear biomolecule 126 so that moieties that are on top at one position of the linear biomolecule 126 are at the opposite side at another position. The sequence of known length may be a sequence of N nucleotides. N may be a Gaussian or other distribution with a probability distribution function, mean and/or a width. The repeated sequence of known length may include one or more recognizable subsequences that may be used to identify the beginning and/or end of the initial template DNA sequence. More than one sequence of known length may be included in the linear biomolecule 126. For example, one sequence of known length may be of length N. Another sequence of known length may be of length N+X. X may be from 1 to 21 nucleotides. N and/or X may be quasirandom, obeying some distribution with a known distribution function, mean and/or width. N and or X may include a known and clearly detectable number of redox nucleotide repeats that may serve as an identifier sequence. For example, the sequence of known length may include 5-10 redox nucleotides in a row or 5-20 alternating redox nucleotides in a row. The corresponding redox-nucleotides become incorporated during the subsequent reactions after ligation and they may be used as checkpoint regions to detect the beginning and the ending of the sequencing of each repetition of the DNA molecule of interest. The identifier sequences may be used to determine which blocks were ligated onto the DNA molecules of interest.

Some fraction of the nucleotides of the copies of the initial template DNA sequence nucleotides may be labeled with a sensing molecule (e.g., label) that may be measured electrochemically. The sensing molecule may be but is not limited to ferrocene, methylene blue, anthracene, quinone, bipyridinium, transitional metal complex, or a molecule including one or more of these moieties. The sensing molecule may produce a measurable electrochemical or electrical signal. The signal may be decomposed into subsequences that are recognized as one or more labeled nucleotides or base pairs. In this way, the system may be used to sequence DNA molecules. By sampling the same strand according to a fixed repetition N, all of the generated orientations of the DNA molecules may be sampled in a known order, thereby allowing for the deciphering of the DNA sequence of the initial template DNA molecule.

In one or more embodiments, a system for detecting more than one portion of a biomolecule during a single pass through a sensor is provided. The system may include a device having at least one electrode and at least one channel sized to receive a linear biomolecule. The linear biomolecule may have at least one label. The linear biomolecule may be a series of repeated units each separated by blocks of known length. The blocks of known length may shift the structure of the linear biomolecule so that at least one moiety of the linear biomolecule on a first side of the linear biomolecule at a first position is on a second side of the linear biomolecule at a second position. The system may also include a readout interface having at least one connection to the device. The readout interface may detect a signal generated by the at least one label and sensed by the at least one electrode.

### Examples:

### Example 1: Simulating a signal pattern

For the simulation shown in Figures 5A and 5B, a sequence of length N=15 that is repeated two times was included in the DNA strand. Figure 5A illustrates a DNA strand, with each Cytosine (C) nucleotide labeled in green. The green label may be sensed with a signal as described analytically earlier. The translucent inset is the same sequence rotated around its axis by 180 degrees. The sequence includes a single labeled site, two adjacent labeled sites, and two nonadjacent labeled sites. The strand was then passed through the nanochannel adjacent to the electrode at a constant velocity of 10 microns per second.

Figure 5B illustrates the simulated signal according to an analytical description described earlier. The parameters of the signal are taken from quantum-mechanical simulations and then passed through a Bessel filter of N=8 and 10 kHz. There is also a baseline noise added to the signal.

As shown in Figures 5A and 5B, each portion has its own fingerprint as a function of the angle of rotation. The single tag, located at 3 and 8 nm in x (300 and 800 us) is a substantially different spectrum than the double adjacent tag, located at 4.5 and 9 nm in x (450 and 900 microseconds in time). These data show that each subsequence has a characteristic fingerprint that depends on the angle of rotation. When the biomolecule is concatemerized, the sensed signal will iteratively sample the curves shown in Figure 5B. These can be used to distinguish the subsequences shown in FIGURE 5A.

### Example 2: Simulating a signal pattern for various redox-type molecules

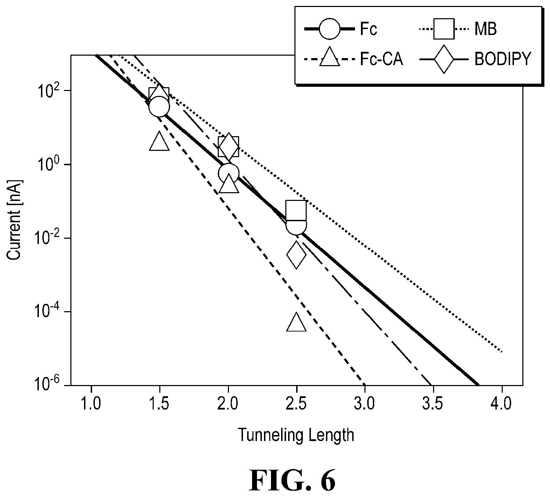

Figure 6 is a graph illustrating calculations of electron transmission through different redox-type molecules as a function of tunneling length. Calculations were done with transiesta using the nonequilibrium Green's function (NEGF) method. Fc = ferrocene; Fc-CA = ferrocene with carboxylic acid end group; MB = methylene blue; and BODIPY = boron dipyrromethene. The lines give approximate fits to the usual exponential model $I ∼ {I}_{0} exp \left(-\frac{2 z}{{z}_{0}}\right)$.

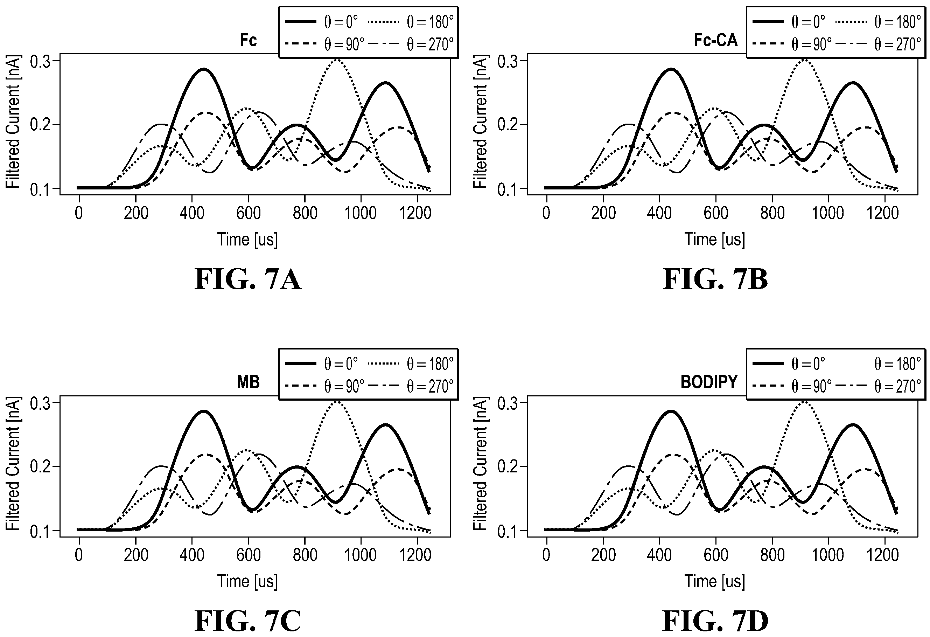

Figures 7A-7D are graphs of profiles for redox-labeled DNA strands unrotated (=0°), or rotated 90°, 180°, or 270°. Rotation-based profiles were recalculated using the parameters outlined in Figure 6. The values of I0 and z0 change the current magnitude, which depends on the tunneling length. Once the renormalized scales are calculated, all molecules have essentially the same profiles. As shown. the noise element was removed for clarity. Figure 7A illustrates the profile for ferrocene. Figure 7B illustrates the profile for ferrocene with a carboxylic acid end group. Figure 7C illustrates the profile for methylene blue. Figure 7D illustrates the profile for boron dipyrromethene (BODIPY).

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

## Claims

1. A system for detecting more than one portion of a biomolecule during a single pass through a sensor comprising:
a device including at least one electrode and at least one channel sized to receive a linear biomolecule having at least one label, the linear biomolecule comprising a series of repeated units each separated by blocks of known length, wherein the blocks of known length shift the structure of the linear biomolecule so that at least one moiety of the linear biomolecule on a first side of the linear biomolecule at a first position is on a second side of the linear biomolecule at a second position; and
a readout interface having at least one connection to the device, wherein the readout interface detects a signal generated by the at least one label and sensed by the at least one electrode.

2. The system of claim 1, wherein the at least one channel is at least one nanochannel having diameter(s) of less than 1 µm.

3. The system of claim 1, wherein the at least one label is at least one redox moiety capable of generating electrical signal(s).

4. The system of claim 1, wherein the signal generated by the at least one label is sensed via proximity(ies) of the at least one label to the at least one electrode at a known potential.

5. The system of claim 1, wherein the device further includes a sensing zone proximate to two electrodes separated by a dielectric of size 1 to 10 nm.

6. The system of claim 1, wherein the linear biomolecule is a polynucleotide.

7. The system of claim 6, wherein the series of repeated units are concatemerized copies of the polynucleotide sequence.

8. The system of claim 7, wherein the blocks of known length are a sequence of length N or a sequence of length N+X.

9. The system of claim 8, wherein the blocks of known length include an identifier sequence.

10. A method of detecting more than one portion of a biomolecule during a single pass through a sensor comprising:
providing a sample including at least one biomolecule, the at least one biomolecule comprising a single unit;
attaching at least one block of known length to the at least one biomolecule;
repeating the single unit of the at least one biomolecule and the at least one block of known length more than one time to generate a linear biomolecule comprising a series of repeated units each separated by the blocks of known length, wherein the blocks of known length shift the structure of the linear biomolecule so that at least one moiety of the linear biomolecule on a first side of the linear biomolecule at a first position is on a second side of the linear biomolecule at another position;
modifying the at least one moiety on the linear biomolecule with at least one label to generate a labeled linear biomolecule;
delivering the labeled linear biomolecule to a device including at least one electrode and at least one channel; and
detecting a signal generated by the at least one label and the at least one electrode.

11. The method of claim 10, wherein the at least one biomolecule is at least one polynucleotide.

12. The method of claim 11, wherein the series of repeated units are concatemerized copies of the at least one polynucleotide.

13. The method of claim 10, wherein the blocks of known length are polynucleotide sequences of length N or length N+X.

14. The method of claim 10, wherein the blocks of known length include an identifier sequence.

15. The method of claim 10, wherein the at least one label is at least one redox moiety capable of generating an electrical signal(s).

16. The method of claim 11, wherein the at least one polynucleotide is fragmented via tagmentation or sonication prior to attaching the blocks of known length to the at least one biomolecule to produce a tunable distribution of lengths of the at least one polynucleotide in the sample.

17. A method of detecting more than one portion of a polynucleotide during a single pass through a sensor comprising:
providing a sample including initial template polynucleotides;
fragmenting the initial template polynucleotides in the sample;
blunt end repairing and tailing the fragmented polynucleotides;
ligating a hairpin adapter onto each end of each of the blunt end repaired and tailed polynucleotides to form a circularized dumbbell structure;
amplifying the circularized dumbbell structure using at least two non-complementary redox-modified dNTPs having distinguishable signals to generate a single-stranded labeled linear polynucleotides having multiple concatemerized copies of the initial template polynucleotides separated by the hairpin adapters;
delivering the labeled linear polynucleotide to a device including at least one electrode and at least one channel; and
detecting a signal generated by the label and the at least one electrode.

18. The method of claim 17, wherein the circularized dumbbell structure is amplified via rolling circle amplification.

19. The method of claim 17, wherein the initial template polynucleotides are fragmented via tagmentation or sonication prior to attaching the hairpin adapters to the at least one biomolecule to produce a tunable distribution of lengths of the at least one polynucleotide in the sample.

20. The method of claim 17, wherein the plurality of the initial template polynucleotides is fragmented via restriction digestion to generate polynucleotides of known length.
